(19) Europäisches Patentamt
European Patent Office
Office européen des brevets

(11) **EP 3 689 820 B1**

(12) **EUROPEAN PATENT SPECIFICATION**

(45) Date of publication and mention of the grant of the patent:
**19.03.2025 Bulletin 2025/12**

(21) Application number: **19154580.5**

(22) Date of filing: **30.01.2019**

(51) International Patent Classification (IPC):
***C01B 33/14*** *(2006.01)* ***C09K 11/02*** *(2006.01)*
***C09K 11/06*** *(2006.01)* ***C09K 11/62*** *(2006.01)*
***C09K 11/70*** *(2006.01)*

(52) Cooperative Patent Classification (CPC):
**C01B 33/14; C09K 11/025; C09K 11/06; C09K 11/703;** C01P 2006/60

(54) **SENSORS COMPRISING MESOPOROUS SILICA PARTICLES**

SENSOREN MIT MESOPORÖSEN SILICIUMDIOXIDPARTIKELN

CAPTEURS COMPRENANT DES PARTICULES DE SILICE MÉSOPOREUSES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**05.08.2020 Bulletin 2020/32**

(73) Proprietor: **Kim, Youngdo**
**Seocho-gu**
**Seoul 06518 (KR)**

(72) Inventors:
- **Lee, Jaeho**
  **66292 Riegelsberg (DE)**
- **Kim, Youngdo**
  **66280 Sulzbach (DE)**

(74) Representative: **Schön, Christoph**
**Dr. Schön, Neymeyr & Partner mbB**
**Bavariaring 26**
**80336 München (DE)**

(56) References cited:
**CN-A- 105 601 938**

- **JUNG BYUNG ET AL: "Molecularly imprinted mesoporous silica particles showing a rapid kinetic binding", vol. 46, no. 21, 1 January 2010 (2010-01-01), pages 3699 - 3701, XP009512489, ISSN: 0577-6058, Retrieved from the Internet <URL:https://doi.org/10.1039/c003173a?nosfx=y> DOI: 10.1039/C003173A**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 689 820 B1

## Description

### TECHNICAL FIELD OF THE INVENTION

The present invention relates to a sensor comprising molecularly imprinted mesoporous silica composite and a luminophore which sensor provides an improved temperature stability and a better storage stability, a process for producing said sensor and the use of said sensor in detecting pollutants, biomarkers, toxic materials or pharmaceutical metabolites.

### BACKGROUND ART OF THE INVENTION

Molecularly imprinted polymers (MIPs) are highly robust crosslinked materials which display good affinity toward a target compound. Usually said MIPs are produced by polymerizing appropriate functional monomers and a crosslinking agent around a target compound (template).

Nevertheless, molecularly imprinted nanoporous polymers which are highly specific to a small organic molecule are rare. For example, the molecularly imprinted polymers described by B. Sellergren et al., "Layer-by-layer grafting of molecularly imprinted polymers via iniferter modified supports", Adv. Mater. 14, (2002), pages 1204-1208 only exhibit low specific molecular recognition properties.

One of the reasons for the low molecular recognition properties of known molecularly imprinted polymers may be seen in the conventional polymerization methods used for producing said molecularly imprinted polymers, as these production methods could not generate molecularly imprinted polymers having an exact and efficient design of the imprinted matrix formed.

Recently, B. M. Jung et al., "Molecularly imprinted mesoporous silica particles showing a rapid kinetic binding", Chem. Commun. 46, (2010), pages 3699-3701 teach that mesoporous silica particles showing an improved sensing property can be produced by forming a recognition site between the pores of the mesoporous silica network and introducing quantum dots into the pores of said mesoporous silica network.

CN 105 601 938 A discloses a preparation method of a pH/temperature double-stimulus-responsive molecularly imprinted polymer comprising the steps of mixing and reacting benzaldehyde-terminated poly(N-isopropylacrylamido) (PNIPAAm) and amino-modified mesoporous silica molecularly imprinted fluorescent polymer to obtain the pH/temperature double-stimulus-responsive molecularly imprinted polymer.

However, also these molecularly imprinted mesoporous silica particles do not show excellent sensitivity and selectivity for specific target molecules as well as a good temperature stability and storage stability.

Therefore, an object of the present invention is to provide molecularly imprinted polymers which can be used as sensor elements having an excellent sensing performance as well as a good temperature stability and storage stability.

### SUMMARY OF THE INVENTION

The present invention provides a sensor comprising molecularly imprinted mesoporous silica comprising a silica matrix and one or more target molecule-binding moieties as defined in claims 1 to 9.

The present invention further provides a process for producing a mesoporous silica comprising a silica matrix and one or more target molecule-binding moieties as defined in claims 10 to 13.

Furthermore, the present invention provides use of a sensor comprising molecularly imprinted mesoporous silica comprising a silica matrix and one or more target molecule-binding moieties as defined in claim 14.

### BRIEF DESCRIPTION OF FIGURES

Fig.1 presents an example of a spectra change determined due to binding of a target molecule to the molecularly imprinted mesoporous silica comprising one or more target molecule-binding moieties claimed according to the present invention.

Fig.2 illustrates schematically the process for producing the molecularly imprinted mesoporous silica comprising one or more target molecule-binding moieties claimed according to the present invention.

Fig.3 presents the structure of a silane of the formula $SiA_3Bm$ comprising a target molecule-binding moiety Bm to be used according to the present invention.

Fig.4 presents a TEM image of the molecularly imprinted mesoporous silica comprising one or more target molecule-binding moieties according to the present invention.

Fig.5 presents small angle X-ray scattering of the molecularly imprinted mesoporous silica comprising one or more target molecule-binding moieties according to the present invention.

Fig.6 presents an emission change determined by binding a target molecule (glyphosate) or competing compounds in various concentrations when using a glyphosate imprinted mesoporous silica containing imidazolium-sulfonate zwitterionic target molecule-binding moieties and blue graphene quantum dots. (a) glyphosate; (b) aminomethyl phosphonic acid (AMPA); (c) glycine.

Fig.7 presents the high selectivity and sensitivity of a molecularly imprinted mesoporous silica comprising

one or more target molecule-binding moieties according to the present invention against glyphosate in emission quenching percent change (a) in the range of measured concentration; (b) in the range between 0.1 nM to 100 nM with concentration log scale in x-axis.

Fig.8 presents an emission change determined by binding a target molecule (3-L-nitro tyrosine) or competing compounds in various concentrations when using a 3-L-nitro tyrosine imprinted mesoporous silica containing imidazolium-sulfonate zwitterionic target molecule-binding moieties and blue graphene quantum dots. (a) 3-L-nitro tyrosine; (b) L-tyrosine; (c) L-tryptophan; (d) L-phenylalanine.

Fig.9 presents an emission change determined by binding a target molecule (glutathione) or competing compounds in various concentrations when using a glutathione disulfide imprinted mesoporous silica containing imidazolium-sulfonate zwitterionic target molecule-binding moieties and blue graphene quantum dots. (a) glutathione disulfide; (b) glutathione.

Fig.10 presents an emission change determined by binding a target molecule (bisphenol S) or competing compounds in various concentrations when using a bisphenol S imprinted mesoporous silica containing imidazolium-sulfonate zwitterionic target molecule-binding moieties and blue graphene quantum dots. (a) bisphenol S; (b) bisphenol A.

Fig.11 presents an emission change determined by binding a target molecule (3-L-nitro tyrosine) in various concentrations when using a 3-L-nitro tyrosine imprinted mesoporous silica containing ammonium-sulfonate zwitterionic target molecule-binding moieties and green graphene quantum dots. (a) $-N(CH_3)_2^+-C_3H_6$-sulfonate (preparation described in Example 5); (b) $-N(CH_3)_2^+-C_4H_8$-sulfonate (Example 6).

Fig.12 presents an emission change determined by binding a target molecule (3-L-nitro tyrosine) in various concentrations when using a 3-L-nitro tyrosine imprinted mesoporous silica containing pyridinium-sulfonate zwitterionic target molecule-binding moieties and green graphene quantum dots. (a) pyridinium-$C_3H_6$-sulfonate (preparation described in Example 7); (b) pyridinium-$C_4H_8$-sulfonate (Example 8).

## *DETAILED DESCRIPTION OF THE INVENTION*

The present invention provides a sensor comprising molecularly imprinted mesoporous silica comprising a silica matrix and one or more target molecule-binding moieties as defined in claim 1.

Usually, the sensor comprising molecularly imprinted mesoporous silica may be present on a substrate. For example, the sensor according to the present invention may be present on glass beads, a chromatographic column or a fibrous or solid support material. However, the sensor according to the present invention may also be used in the form of nanoparticles in the absence of a support material.

The sensor according to the present invention provides a very high selectivity for target molecules usually having a molecule size in a range of from 0.01 to 100 nm, preferably in a range of from 0.1 to 50 nm, more preferably in a range of from 1 to 25 nm.

According to the present invention it was found that the sensor according to the present invention provides an excellent sensitivity for selectively detecting a specific target molecule. For example, when using ELISA as a detecting technique the detection limit of ELISA usually is approximately 50 ppt.

In contrast, when using a sensor according to the present invention, the detection limit is approximately 2 to 3 times lower than when using ELISA.

According to the present invention the silica matrix of the sensor comprises one or more target molecule-binding moieties formed of $-(CH_2)_n-X^+-(CH_2)_m-AN^-$ groups, wherein $X^+$ represents a cationic $-NR_2^+-$ group or a cationic heterocyclic group comprising at least one N atom, R represents $(C_1-C_4)$ alkyl groups, m and n irrespectively of each other represent an integer of from 1 to 6 and $AN^-$ represents $-COO^-$ or $-SO_3^-$, wherein in said target-molecule-binding moieties the parameter $X^+$ of a cationic heterocyclic group comprising at least one N atom represents one of the following groups:

alkyl pyrrolidinium:

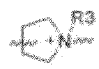

imidazolinium:

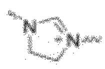

imidazolium:

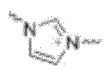

triazolium:

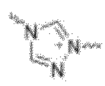

piperazinium:

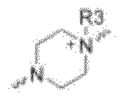

pyridinium:

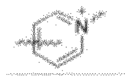

and

triazinium:

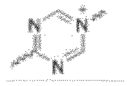

wherein R3 represents $C_rH_{2r+1}$, wherein r represents an integer of from 1 to 4.

In a preferred embodiment, in said target molecule-binding moieties the parameter $X^+$ represents an imidazolinium group as shown above, which is bound to the two carbon atoms via its two N ring atoms.

Furthermore, in said target molecule-binding moieties preferably the parameter $AN^-$ represents $-SO_3^-$, n represents an integer of 1 to 4 and/or m represents an integer of 3 or 4.

In an even more preferred embodiment in said target molecule-binding moieties the parameter $X^+$ represents an imidazolinium group which is bound to the two carbon atoms via its N atoms, $AN^-$ represents $-SO_3^-$, n represents an integer of 1 to 4 and/or m represents an integer of 3 or 4.

Furthermore, the signal element is a luminophore, and preferably a fluorescent material or a chemiluminescent material.

In an even more preferred embodiment, in said target molecule-binding moieties the parameter $X^+$ represents an $-NR_2^+$ group as shown above, which is bound to the N atom, wherein each of the two R residues represents a methyl group.

Furthermore, in a preferred embodiment in said target molecule-binding moieties preferably the parameter $AN^-$ represents $-SO_3^-$, n represents an integer of 1 to 4 and/or m represents an integer of 3 or 4.

In an even more preferred embodiment, in said target molecule-binding moieties the parameter $X^+$ represents an $-NR_2^+$ group as shown above, which is bound to the N atom, wherein each or the two R residues represents a methyl group, $AN^-$ represents $-SO_3^-$, n represents an integer of 1 to 4 and/or m represents an integer of 3 or 4.

Furthermore, the signal element is a luminophore, and preferably a fluorescent material or a chemiluminescent material.

In an even more preferred embodiment, in said target molecule-binding moieties the parameter $X^+$ represents a pyridinium group as shown above, which is bound to the two carbon atoms via its ring C atom in 4-position and its ring N atom.

Furthermore, in said target molecule-binding moieties preferably the parameter $AN^-$ represents $-SO_3^-$, n represents an integer of 1 to 4 and/or m represents an integer of 3 or 4.

In an even more preferred embodiment, in said target molecule-binding moieties the parameter $X^+$ represents a pyridinium group as shown above, which is bound to the two carbon atoms via its ring C atom in 4-position and its ring N atom, $AN^-$ represents $-SO_3^-$, n represents an integer of 1 to 4 and/or m represents an integer of 3 or 4.

Furthermore, the signal element is a luminophore, and preferably a fluorescent material or a chemiluminescent material.

In a more preferred embodiment as a luminophore quantum dot, more preferably graphene quantum dot, carbon quantum dot, semiconductor II-VI quantum dot or semiconductor III-V quantum dot, even more preferably InP/ZnS or graphene quantum dot is used.

According to the present invention as a sensor molecularly imprinted mesoporous silica as defined above is used to analyze substrates sensitively. Hereby, preferably a fluorescent material, more preferably quantum dots may be used as a signal transducer in order to determine on a sub-nanomolar concentration level the fluorescence change behavior.

Emission from the signal transducer (for example when using quantum dots as a fluorescent signal material) changes the fluorescence intensity by rebinding of the substrate in aqueous media. The change was analyzed by calculating the percentage of intensity change for both quenching (Q%) and enhancing (E%):

$$\text{Q\% or E\%} = |F_0 - F|/F_0 \times 100$$

where $F_0$ and $F$ represents the emission intensities from non-binding and substrate binding values, respectively.

The present invention further provides a process for producing a mesoporous silica comprising a silica matrix and one or more target molecule-binding moieties as defined in claim 10.

Said sol-gel reaction usually is well known in the respective field of the art. Nevertheless, we will describe said sol-gel reaction in more detail below.

In general, as it is well known to a person skilled in the art during the sol-gel reaction tetraalkoxy silane compounds, such as tetramethoxy silane or tetraethoxy silane, are hydrolyzed to form a colloidal solution (a sol) and further polycondensed in the presence of the silane compound of the formula $SiA_3Bm$ as outlined above, said sol-gel reaction being carried out in the presence of the respective target molecule to be detected so as to form highly crosslinked silica materials (gels) comprising one or more target molecule-binding moieties formed of

$-(CH_2)_n-X^+-(CH_2)_m-AN^-$ groups. Said sol-gel reaction usually is carried out in the additional presence of a surfactant such as a quaternary ammonium compound, preferably a trimethyl ($C_{14}$-$C_{24}$-alkyl) ammonium compound, such as trimethyl hexadecyl ammonium chloride or bromide or trimethyl octadecyl ammonium chloride or bromide.

Furthermore, the sol-gel reaction usually is carried out under alkaline pH conditions by adding for example a sodium hydroxide solution to the reaction mixture to be subjected to the sol-gel reaction.

Thereafter, the precipitated highly cross-linked mesoporous silica imprinting matrix material is usually removed from the reaction mixture for example by filtration, washed with water, preferably distilled water, and/or an organic solvent such as ethanol and/or acetone, and dried in a vacuum oven.

Thereafter, the highly cross-linked mesoporous silica imprinting matrix material comprising one or more target molecule-binding moieties formed of $-(CH_2)_n-X^+-(CH_2)_m-AN^-$ groups usually is treated with an extractant so as to remove the target molecules. Such a removal step for example can be carried out by heating a highly cross-linked mesoporous silica imprinting matrix material comprising one or more target molecule-binding moieties formed of $-(CH_2)_n-X^+-(CH_2)_m-AN^-$ groups in a solvent mixture comprising water and one or more organic solvents such as 1,4 dioxane for several hours and thereafter washing and drying the final highly cross-linked mesoporous silica imprinting matrix material comprising one or more target molecule-binding moieties formed of $-(CH_2)_n-X^+-(CH_2)_m-AN^-$ groups which matrix material does not contain the respective target molecule any longer.

According to the present invention said sol-gel reaction produces a highly cross-linked mesoporous silica imprinting matrix that allows for delicate recognition site generation through molecular imprinting methodology. This makes it unique among molecularly imprinted nanomaterials which usually do not afford highly specific solid or homogeneous imprinted cavities for the specific target molecules. According to the present invention selective grafting on a mesoporous silica matrix promotes facile chemical group functionalization. This grafting joins the chemical moieties to the pore surface and/or framework of silica matrix, thereby these groups can be used as highly selective sensor for recognizing specific target molecules.

According to the present invention a substrate binding silica precursor, $SiA_3Bm$, wherein A is a halide or alkoxy group, for example a chlorine group, a bromine group, a methoxy group, an ethoxy group, an n-propoxy group, an iso-propoxy group, a n-butyloxy group, an iso-butyloxy group or a tert.-butoxy group, a Bm group represents a target molecule-binding moiety formed of $-(CH_2)_n-X^+-(CH_2)_m-AN^-$ groups as defined in claim 10, -, is added to a tetraalkoxy silane so as to prepare molecularly imprinted mesoporous silica comprising one or more target molecule-binding moieties formed of $-(CH_2)_n-X^+-(CH_2)_m-AN^-$ groups.

With respect to the preparation of a silane of the formula $SiA_3Bm$, said starting material may be prepared by nucleophilic substitution reactions ($S_N$) or ring opening reactions. For the preparation by $S_N$ reaction, usually mono halogenated alkanoic acids are refluxed with $SiA_3-(CH_2)_n-X$, wherein X represents tertiary amine, $-NR_2$, or heterocyclic group containing at least one N atom dissolved in absolute ethanol or methanol in the presence of a base such as triethyl amine. The products are separated by solvent evaporation after filtration of the by-product (= ammonium halides). The products can be used without further purification. The sulfonate group containing Bm can be prepared by heating under reflux in absolute ethanol and methanol mixture solution of the tertiary amine or heterocyclic group containing $SiA_3-(CH_2)_n-X$ and cyclic sultones, e.g. 1,4-butane sultone, by sultone ring opening. The molar ratio of the reactants usually is around 1:1 and the products can be separated by solvent evaporation without needing further purification steps.

As already outlined above, during the formation of mesoporous silica matrix, the target molecule-binding moiety or moieties is/are implanted in the framework of the matrix. Thereafter, an extraction process detaches only substrate exposed to the surface and the binding moiety/moieties of the complex is/are intact in the vicinity of created hollows having fixed conformation. Selectivity enhancement towards substrates may be attributed to the cooperation between binding site specificity and interaction with the complexing functional groups, which make a big difference to the rebinding property even from structurally similar molecules sterically capable of fitting into the cavities. Unlike QD colloidal suspension, encapsulated multiple QD particles have a regular layout in confined volume. They are placed in channels of mesoporous silica. Simple change of the imprinting substrates produces different chemical targeted sensing probes in the same preparation procedure so that the sensor can be applied to detect a large number of small target molecules with high versatility.

After the target molecule was removed from the highly cross-linked mesoporous silica imprinting matrix material, according to the present invention a signal element usually is introduced into the highly cross-linked mesoporous silica imprinting matrix material.

As outlined above, as a signal element a luminophore, preferably a fluorescent material or a chemiluminescent material, more preferably graphene quantum dot, carbon quantum dot, semiconductor II-VI quantum dot or semiconductor III-V quantum dot, even more preferably InP/ZnS or graphene quantum dot is used.

In order to introduce the signal element into the highly crosslinked mesoporous silica imprinting matrix material usually a signal element is dissolved or suspended in an organic solvent and thereafter added to the highly crosslinked mesoporous silica imprinting matrix

material, whereupon the mixture is stirred for up to several hours. Usually the mass ratio of signal element to the highly crosslinked mesoporous silica imprinting matrix material in the mixture is in a range of from 1:10 to 1:200, more preferably 1:50 to 1:150 based on the weight of the two components.

Thereafter, the highly crosslinked mesoporous silica imprinting matrix material comprising the signal element is removed from the mixture for example by filtration, washed with water and/or organic solvent and dried so as to produce the final highly crosslinked mesoporous silica imprinting matrix material to be used as the sensor according to the present invention.

The presence of the signal element in the final highly crosslinked mesoporous silica imprinting matrix material can be verified for example using a small angle X-ray scattering (SAXS) or high resolution transmission electrode microscope (HR-TEM).

Furthermore, the present invention provides use of a sensor comprising molecularly imprinted mesoporous silica comprising a silica matrix and one or more target molecule-binding moieties as defined in claim 14.

**Examples**

The following examples should not limit the disclosure of the present application and are only provided as additional explanation how to carry out the present invention.

In more detail, in the following examples, four different target compounds were used in the synthesis of a sensor according to the present invention, i.e., glyphosate (pesticide), 3-L-nitrotyrosine, glutathione disulfide (biomarkers), and bisphenol S (plastic additive). Rebinding induces quenching of emission by means of fluorescence resonance energy transfer (FRET) except glutathione disulfide regardless of the type of quantum dots. They respond sensitively in the concentration as low as 0.1nM only for all the imprinted molecules. The intensity changes for the substrates is distinguishable from the competing chemicals which are structurally very similar as well as charge of the molecules. Even 1000 times higher concentration, 100 nM, of competitors do not change the signal as much as that of target substrate in 0.1 nM. The probe materials, QD-Bm-MIMS, are highly selective (Fig.6, Fig.8, Fig.9, Fig.10, Fig. 11, and Fig. 12). For the glyphosate measurement, the percent quenching of the emission is proportional to the log scale of the concentration especially in very low concentrations which implies they are able to assay analytes in ppt levels (Fig.7).

Example 1: Glyphosate imprinted mesoporous silica particles with 3-(4-sulfonatobutyl)-1-[3-(triethoxysilyl) propyll-1H-imidazol-3-ium

*Synthesis of glyphosate-imprinted mesoporous silica nanoparticles (Bm-MIMS, glyphosate)*

An aqueous NaOH solution (1.0 M, 7.0 mL, Sigma Aldrich) was added to a solution of hexadecyltrimethylammonium bromide (0.94 g, 99%, Sigma Aldrich) in distilled water (480 mL). After adding glyphosate (50 mg, 96%, Sigma Aldrich) and 3-(4-sulfonatobutyl)-1-[3-(triethoxysilyl)propyl]-1H-imidazol-3-ium (70 mg), tetraethyl orthosilicate (5.12 g, 99%, Sigma Aldrich) was added dropwise. The reaction mixture was subsequently stirred at 70 0C for 3 h. The precipitated product was filtered, washed with distilled water, ethanol and acetone, and dried in vacuum oven at 60 0C for 2 days. The product (2.0 g) was refluxed in a mixed solution of HCl (35 wt%, 10 g), distilled water (50 g) and 1,4-dioxane (150 g) at 110 0C for 24 h. The product was isolated by filtration, washed with distilled water, ethanol and acetone, and dried in vacuo at 60 $^0$C for 3 days.

*Synthesis of the graphene or InP/ZnS quantum dot-encapsulated glyphosate imprinted mesoporous silica nanoparticles (QD-Bm-MIMS, glyphosate)*

Graphene quantum dots (5 mL, 1 mg mL-1 in $H_2O$, Sigma Aldrich) and InP/ZnS quantum dots (1 mL, 5 mg mL-1 in toluene, Sigma Aldrich) were added to a suspension of Bm-MIMS (500 mg) in $H_2O$ and toluene, respectively. The mixture was stirred for 3 h and isolated by filtration, washed with distilled water or toluene and dried in vacuo at 30 $^0$C for 5 days. Mesoporous structure and QD encapsulation were confirmed by small angle X-ray scattering (SAXS) and high resolution transmission electrode microscope (HR-TEM). (Fig.4 and Fig.5)

*Glyphosate detection*

All of the analytical solutions of glyphosate and its analogues, aminomethylphosphonic acid (AMPA), glycine, and glutamine were prepared by dissolving them in deionized water. The diluted QD-Bm-MIMS suspension (2 mg $mL^{-1}$) was prepared using ethanol. In assessment for the sensitivity and single-component selectivity test QD-Bm-MIMS ethanol suspension (50 μL) and different concentration of analytes (2.0 mL) were mixed and stirred in each vial for 5 mins, and then fluorescence spectra were measured at 350 nm excitation wavelength. The intensities of emission were recorded at λmax of InP/ZnS QD (532 nm), blue GQD (451 nm), and green GQD (530 nm)

Example 2: 3-L-nitrotyrosine imprinted mesoporous silica particles

All the procedure is the same to the example 1 except using 3-L-nitro tyrosine in the particle synthesis and L-tyrosine, L-phenylalanine, and L-tryptophan as competing chemicals.

Example 3: Glutathione disulfide imprinted mesoporous silica particles

All the procedure is the same to the example 1 except using glutathione disulfide in the particle synthesis and glutathione as competing chemicals.

Example 4: Bisphenol S imprinted mesoporous silica particles

All the procedure is the same to the example 1 except using bisphenol S in the particle synthesis and bisphenol A as competing chemicals.

Example 5: 3-L-nitrotyrosine imprinted mesoporous silica particles with 3-{dimethyl[3-(trimethoxysilyl)propyl]azaniumyl}propane-1-sulfonate

All the procedure is the same to the example 2 except using 3-{dimethyl[3-(trimethoxysilyl)propyl]azaniumyl}propane-1-sulfonate in the particle synthesis instead of 3-(4-sulfonatobutyl)-1-[3-(triethoxysilyl)propyl]-1H-imidazol-3-ium.

Example 6: 3-L-nitrotyrosine imprinted mesoporous silica particles with 4-{dimethyl[3-(trimethoxysilyl)propyl]azaniumyl}butane-1-sulfonate

All the procedure is the same to the example 2 except using 4-{dimethyl[3-(trimethoxysilyl)propyl]azaniumyl}butane-1-sulfonate in the particle synthesis instead of 3-(4-sulfonatobutyl)-1-[3-(triethoxysilyl)propyl]-1H-imidazol-3-ium.

Example 7: 3-L-nitrotyrosine imprinted mesoporous silica particles with 1-(4-sulfonatopropyl)-4-[2-(trimethoxysilyl)ethyl]pyridin-1-ium

All the procedure is the same to the example 2 except using 1-(4-sulfonatopropyl)-4-[2-(trimethoxysilyl)ethyl]pyridin-1-ium in the particle synthesis instead of 3-(4-sulfonatobutyl)-1-[3-(triethoxysilyl)propyl]-1H-imidazol-3-ium.

Example 8: 3-L-nitrotyrosine imprinted mesoporous silica particles with 1-(4-sulfonatobutyl)-4-[2-(trimethoxysilyl)ethyl]pyridin-1-ium

All the procedure is the same to the example 2 except using 1-(4-sulfonatobutyl)-4-[2-(trimethoxysilyl)ethyl]pyridin-1-ium in the particle synthesis instead of 3-(4-sulfonatobutyl)-1-[3-(triethoxysilyl)propyl]-1H-imidazol-3-ium.

## Claims

1. A sensor comprising molecularly imprinted mesoporous silica comprising a silica matrix and one or more target molecule-binding moieties formed of $-(CH_2)_n-X^+-(CH_2)_m-AN^-$ groups, wherein $X^+$ represents a cationic $-NR_2^+-$ group or a cationic heterocyclic group comprising at least one N atom wherein the cationic heterocyclic group comprising at least one N atom is selected from the group consisting of

alkyl pyrrolidinium:

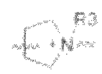

,

imidazolinium:

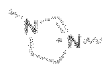

imidazolium:

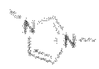

triazolium:

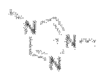

piperazinium:

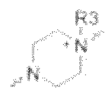

pyridinium:

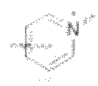

and
triazinium:

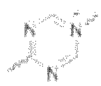

,

wherein R3 represents $C_rH_{2r+1}$, wherein r represents an integer of from 1 to 4, R represents

$(C_1-C_4)$ alkyl groups, m and n irrespectively of each other represent an integer of from 1 to 6 and $AN^-$ represents $-COO^-$ or $-SO_3^-$, said sensor further comprising a luminophore as a signal element.

**2.** The sensor according to claim 1, wherein n represents an integer of 1 to 4, preferably 2 or 3 and m represents an integer of 3 or 4.

**3.** The sensor according to claim 1 or 2, wherein $AN^-$ represents $-SO_3^-$.

**4.** The sensor according to any one of claims 1 to 3, wherein $X^+$ represents a heterocyclic group.

**5.** The sensor according to any one of claims 1 to 4, wherein $X^+$ represents an imidazole group.

**6.** The sensor according to any one of claims 1 to 5, wherein $X^+$ represents an imidazole group bound through the two N atoms to respective carbon atoms.

**7.** The sensor according to any one of claims 1 to 6, wherein graphene quantum dot, carbon quantum dot, semiconductor II-VI quantum dot or semiconductor III-V quantum dot is used as a signal element.

**8.** The sensor according to any one of claims 1 to 7, wherein semiconductor quantum dot as a luminophore is present in the pores of the molecularly imprinted mesoporous silica.

**9.** The sensor according to any one of claims 1 to 7, wherein graphene quantum dot as a luminophore is present in the pores of the molecularly imprinted mesoporous silica.

**10.** A process for producing a mesoporous silica comprising a silica matrix, a luminophore as a signal element and one or more target molecule-binding moieties formed of $-(CH_2)_n-X^+-(CH_2)_m-AN^-$ groups, wherein $X^+$ represents a cationic $-NR_2^+-$ group or a cationic heterocyclic group comprising at least one N atom wherein the cationic heterocyclic group comprising at least one N atom is selected from the group consisting of

alkyl pyrrolidinium:

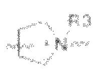

,

imidazolinium:

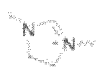

imidazolium:

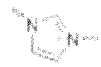

triazolium:

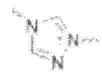

piperazinium:

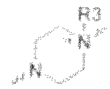

pyridinium:

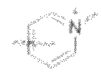

and

triazinium:

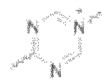

,

wherein R3 represents $C_rH_{2r+1}$, wherein r represents an integer of from 1 to 4, R represents $(C_1-C_4)$ alkyl groups, m and n irrespectively of each other represent an integer of from 1 to 6 and $AN^-$ represents $-COO^-$ or $-SO_3^-$, said process comprising a step of subjecting a tetraalkoxy silane and a silane of the formula $SiA_3Bm$ comprising a target molecule-binding moiety Bm formed of a $-(CH_2)_n-X^+-(CH_2)_m-AN^-$ group, wherein A represents a halide ion or an alkoxy group of the formula $-OC_xH_{2x+1}$, wherein x represents an integer of from 1 to 4, X represents a cationic $-NR_2^+-$ group or a cationic heterocyclic group comprising at least one N atom, R represents $(C_1-C_4)$ alkyl groups, m and n irrespectively of each other represent an integer of from 1 to 6 and $AN^-$ represents $-COO^-$ or $-SO_3^-$, to a sol-gel reaction in the presence of the respective target molecule or target molecules.

**11.** The process according to claim 10, wherein the sol-gel reaction is carried out in the presence of a surfactant.

**12.** The process according to any one of claims 10 and 11, wherein tetraethoxy silane is used as the tetraalkoxy silane.

**13.** The process according to any one of claims 10 to 12,

wherein hexadecyltrimethylammonium bromide is used as the surfactant.

**14.** Use of a sensor comprising molecularly imprinted mesoporous silica comprising a silica matrix and one or more target molecule-binding moieties formed of $-(CH_2)_n-X^+-(CH_2)_m-AN^-$ groups, wherein $X^+$ represents a cationic - $NR_2^+$- group or a cationic heterocyclic group comprising at least one N atom wherein the cationic heterocyclic group comprising at least one N atom is selected from the group consisting of

alkyl pyrrolidinium:

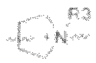

,

imidazolinium:

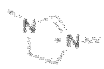

imidazolium:

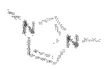

triazolium:

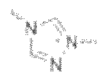

piperazinium:

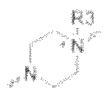

pyridinium:

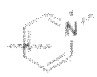

and
triazinium:

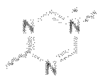

,

wherein R3 represents $C_rH_{2r+1}$, wherein r represents an integer of from 1 to 4, R represents $(C_1-C_4)$ alkyl groups, m and n irrespectively of each other represent an integer of from 1 to 6 and $AN^-$ represents $-COO^-$ or $-SO_3^-$, said sensor further comprising a luminophore, as a detector of a pollutant, of a biomarker, or of a pharmaceutical metabolite.

## Patentansprüche

**1.** Sensor, der molekular geprägtes mesoporöses Siliciumdioxid umfasst, das eine Siliciumdioxidmatrix und eine oder mehrere Zielmolekül-bindende Einheiten, die aus - $(CH_2)_n-X^+-(CH_2)_m-AN^-$-Gruppen gebildet sind, umfasst, wobei $X^+$ für eine kationische $-NR_2{}^+$-Gruppe oder eine kationische heterozyklische Gruppe, die mindestens ein N-Atom umfasst, steht, wobei die kationische heterozyklische Gruppe, die mindestens ein N-Atom umfasst, aus der Gruppe ausgewählt ist, die aus den folgenden Verbindungen besteht:

Alkylpyrrolidinium:

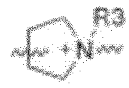

Imidazolinium:

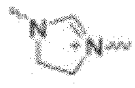

Imidazolium:

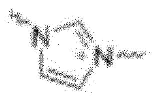

Triazolium:

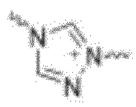

Piperazinium:

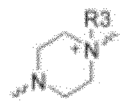

Pyridinium:

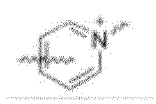

und
Triazinium:

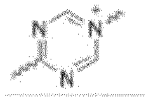 ,

wobei R3 für $C_rH_{2r+1}$ steht, wobei r für eine ganze Zahl von 1 bis 4 steht, R für $(C_1-C_4)$-Alkylgruppen steht, m and n unabhängig voneinander für eine ganze Zahl von 1 bis 6 stehen und $AN^-$ für $-COO^-$ oder $-SO_3^-$ steht, wobei der Sensor des Weiteren ein Luminophor als Signalelement umfasst.

**2.** Sensor gemäß Anspruch 1, wobei n für eine ganze Zahl von 1 bis 4, vorzugsweise 2 oder 3, steht und m für eine ganze Zahl von 3 oder 4 steht.

**3.** Sensor gemäß Anspruch 1 oder Anspruch 2, wobei $AN^-$ für $-SO_3^-$ steht.

**4.** Sensor gemäß einem der Ansprüche 1 bis 3, wobei $X^+$ für eine heterozyklische Gruppe steht.

**5.** Sensor gemäß einem der Ansprüche 1 bis 4, wobei $X^+$ für eine Imidazolgruppe steht.

**6.** Sensor gemäß einem der Ansprüche 1 bis 5, wobei $X^+$ für eine Imidazolgruppe, die über die beiden N-Atome an die jeweiligen Kohlenstoffatome gebunden ist, steht.

**7.** Sensor gemäß einem der Ansprüche 1 bis 6, wobei ein Graphenquantenpunkt, ein Kohlenstoffquantenpunkt, ein Halbleiter-II-VI-Quantenpunkt oder ein Halbleiter-III-V-Quantenpunkt als Signalelement verwendet wird.

**8.** Sensor gemäß einem der Ansprüche 1 bis 7, wobei ein Halbleiterquantenpunkt als Luminophor in den Poren des molekular geprägten mesoporösen Siliciumdioxids vorhanden ist.

**9.** Sensor gemäß einem der Ansprüche 1 bis 7, wobei ein Graphenquantenpunkt als Luminophor in den Poren des molekular geprägten mesoporösen Siliciumdioxids vorhanden ist.

**10.** Verfahren zur Herstellung eines mesoporösen Siliciumdioxids, das eine Siliciumdioxidmatrix, ein Luminophor als Signalelement und eine oder mehrere Zielmolekül-bindende Einheiten, die aus $-(CH_2)_n-X^+-(CH_2)_m-AN^-$-Gruppen gebildet sind, umfasst, wobei $X^+$ für eine kationische $-NR_2^+$-Gruppe oder eine kationische heterozyklische Gruppe, die mindestens ein N-Atom umfasst, steht, wobei die kationische heterozyklische Gruppe, die mindestens ein N-Atom umfasst, aus der Gruppe ausgewählt ist, die aus den folgenden Verbindungen besteht:

Alkylpyrrolidinium:

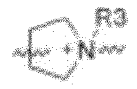

Imidazolinium:

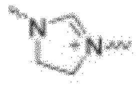

Imidazolium:

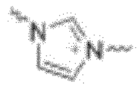

Triazolium:

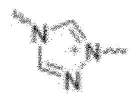

Piperazinium:

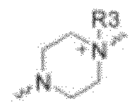

Pyridinium:

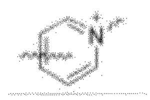

und

Triazinium:

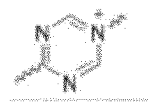 ,

wobei R3 für $C_rH_{2r+1}$ steht, wobei r für eine ganze Zahl von 1 bis 4 steht, R für $(C_1-C_4)$-Alkylgruppen steht, m and n unabhängig voneinander für eine ganze Zahl von 1 bis 6 stehen und $AN^-$ für $-COO^-$ oder $-SO_3^-$ steht, wobei das Verfahren einen Schritt umfasst, bei dem ein Tetraalkoxysilan und ein Silan der Formel $SiA_3Bm$, das eine Zielmolekül-bindende Einheit Bm, die aus einer $-(CH_2)_n-X^+-(CH_2)_m-AN^-$-Gruppe gebildet ist, umfasst, wobei A für ein Halogenidion oder eine Alkoxygruppe der Formel $-OC_xH_{2x+1}$ steht, wobei x für eine ganze Zahl von 1 bis 4 steht, X für eine kationische $-NR_2^+$-Gruppe oder eine kationische heterozyklische Gruppe, die

mindestens ein N-Atom umfasst, steht, R für $(C_1\text{-}C_4)$-Alkylgruppen steht, m und n unabhängig voneinander für eine ganze Zahl von 1 bis 6 stehen und $AN^-$ für $-COO^-$ oder $-SO_3^-$ steht, einer Sol-Gel-Reaktion in Gegenwart des jeweiligen Zielmoleküls oder der jeweiligen Zielmoleküle unterzogen werden.

**11.** Verfahren gemäß Anspruch 10, wobei die Sol-Gel-Reaktion in Gegenwart eines Tensids durchgeführt wird.

**12.** Verfahren gemäß einem der Ansprüche 10 und 11, wobei Tetraethoxysilan als das Tetraalkoxysilan verwendet wird.

**13.** Verfahren gemäß einem der Ansprüche 10 bis 12, wobei Hexadecyltrimethylammoniumbromid als das Tensid verwendet wird.

**14.** Verwendung eines Sensors, der molekular geprägtes mesoporöses Siliciumdioxid umfasst, das eine Siliciumdioxidmatrix und eine oder mehrere Zielmolekül-bindende Einheiten, die aus $-(CH_2)_n\text{-}X^+\text{-}(CH_2)_m\text{-}AN^-$-Gruppen gebildet sind, umfasst, wobei $X^+$ für eine kationische $-NR_2^+$-Gruppe oder eine kationische heterozyklische Gruppe, die mindestens ein N-Atom umfasst, steht, wobei die kationische heterozyklische Gruppe, die mindestens ein N-Atom umfasst, aus der Gruppe ausgewählt ist, die aus den folgenden Verbindungen besteht:

Alkylpyrrolidinium:

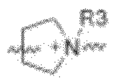

Imidazolinium:

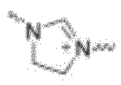

Imidazolium:

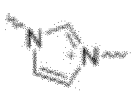

Triazolium:

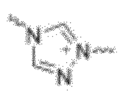

Piperazinium:

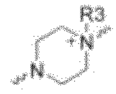

Pyridinium:

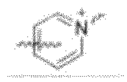

und

Triazinium:

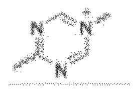

,

wobei R3 für $C_rH_{2r+1}$ steht, wobei r für eine ganze Zahl von 1 bis 4 steht, R für $(C_1\text{-}C_4)$-Alkylgruppen steht, m and n unabhängig voneinander für eine ganze Zahl von 1 bis 6 stehen und $AN^-$ für $-COO^-$ oder $-SO_3^-$ steht, wobei der Sensor des Weiteren ein Luminophor als einen Detektor eines Schadstoffs, eines Biomarkers oder eines pharmazeutischen Metaboliten umfasst.

## Revendications

**1.** Capteur comprenant de la silice mésoporeuse imprimée de manière moléculaire comprenant une matrice de silice et une ou plusieurs fractions de liaison à une molécule cible formées des groupes $-(CH_2)_n\text{-}X^+\text{-}(CH_2)_m\text{-}AN^-$, $X^+$ représentant un groupe $-NR_2^+$- cationique ou un groupe hétérocyclique cationique comprenant au moins un atome N, le groupe hétérocyclique cationique comprenant au moins un atome N étant choisi dans le groupe constitué par

le pyrrolidinium d'alkyle

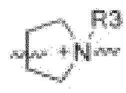

,

l'imidazolinium

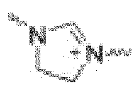

l'imidazolium :

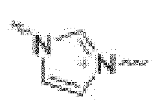

le triazolium :

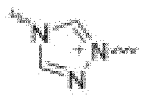

le pipérazinium :

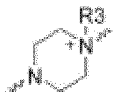

le pyridinium :

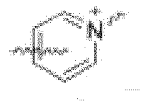

et
le triazinium :

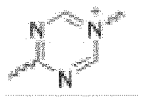

,

dans lesquels R3 représente $CH_{2r+1}$, r représentant un nombre entier de 1 à 4, R représentant des groupes alkyle ($C_1$ à $C_4$), m et n irrespectivement l'un de l'autre représentant un nombre entier de 1 à 6 et $AN^-$ représentant $-COO^-$ ou $-SO_3^-$, ledit capteur comprenant en outre un luminophore comme élément de signal.

**2.** Capteur selon la revendication 1, dans lequel n représente un nombre entier de 1 à 4, de préférence 2 ou 3 et m représente un nombre entier de 3 ou 4.

**3.** Capteur selon la revendication 1 ou 2, dans lequel $AN^-$ représente $-SO_3^-$.

**4.** Capteur selon l'une quelconque des revendications 1 à 3, dans lequel $X^+$ représente un groupe hétérocyclique.

**5.** Capteur selon l'une quelconque des revendications 1 à 4, dans lequel $X^+$ représente un groupe imidazole.

**6.** Capteur selon l'une quelconque des revendications 1 à 5, dans lequel $X^+$ représente un groupe imidazole lié par les deux atomes N aux atomes de carbone respectifs.

**7.** Capteur selon l'une quelconque des revendications 1 à 6, dans lequel le point quantique de graphène, le point quantique de carbone, le point quantique de semiconducteur II-VI ou le point quantique de semiconducteur III-V est utilisé comme élément de signal.

**8.** Capteur selon l'une quelconque des revendications 1 à 7, dans lequel le point quantique de semiconducteur comme luminophore est présent dans les pores de la silice mésoporeuse imprimée de manière moléculaire.

**9.** Capteur selon l'une quelconque des revendications 1 à 7, dans lequel le point quantique de graphène comme luminophore est présent dans les pores de la silice mésoporeuse imprimée de manière moléculaire.

**10.** Procédé de production d'une silice mésoporeuse comprenant une matrice de silice, un luminophore comme élément de signal et une ou plusieurs fractions de liaison à une molécule cible formée des groupes $-(CH_2)_n-X^+-(CH_2)_m-AN^-$, $X^+$ représentant un groupe $-NR_2^+-$ cationique ou un groupe hétérocyclique cationique comprenant au moins un atome N, le groupe hétérocylique cationique comprenant au moins un atome N étant choisi dans le groupe constitué par

le pyrrolidinium d'alkyle

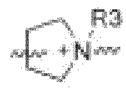

,

l'imidazolinium :

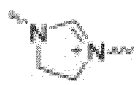

l'imidazolium :

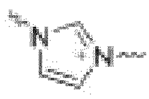

le triazolium :

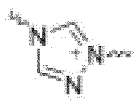

le pipérazinium :

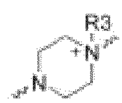

le pyridinium :

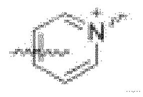

et
le triazinium :

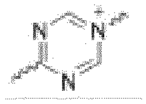

,

dans lesquels R3 représente $CH_{2r+1}$, r représentant un nombre entier de 1 à 4, R représentant des groupes alkyle ($C_1$ à $C_4$), m et n irrespectivement l'un de l'autre représentant un nombre entier de 1 à 6 et $AN^-$ représentant $-COO^-$ ou - $SO_3^-$, ledit procédé comprenant une étape de soumission d'un silane tétraalcoxy et d'un silane de formule $SiA_3Bm$ comprenant une fraction de liaison à une molécule cible Bm formée d'un groupe $-(CH_2)_n-X^+-(CH_2)_m-AN^-$, A représentant un ion halogénure ou un groupe alcoxy de formule-$OC_xH_{2x+1}$, x représentant un nombre entier de 1 à 4, X représentant un groupe $-NR_2^+-$ cationique ou un groupe hétérocyclique cationique comprenant au moins un atome N, R représentant des groupes alkyle ($C_1$ à $C_4$), m et n irrespectivement l'un de l'autre représentant un nombre entier de 1 à 6 et $AN^-$ représentant $-COO^-$ ou $-SO_3^-$, à une réaction sol-gel en présence de la molécule cible ou des molécules cibles respectives.

**11.** Procédé selon la revendication 10, dans lequel la réaction sol-gel est réalisée en présence d'un tensioactif.

**12.** Procédé selon l'une quelconque des revendications 10 et 11, dans lequel le silane tétraéthoxy est utilisé comme le silane tétraalcoxy.

**13.** Procédé selon l'une quelconque des revendications 10 à 12, dans lequel le bromure d'hexadécyltriméthylammonium est utilisé comme tensioactif.

**14.** Utilisation d'un capteur comprenant de la silice mésoporeuse imprimée de manière moléculaire comprenant une matrice de silice et une ou plusieurs fractions de liaison à une molécule cible formée de groupes $-(CH_2)_n-X^+-(CH_2)_m-AN^-$, X représentant un groupe $-NR_2^+-$ cationique ou un groupe hétérocyclique cationique comprenant au moins un atome N, le groupe hétérocyclique cationique comprenant au moins un atome N étant choisi dans le groupe constitué par

le pyrrolidinium d'alkyle

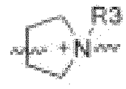

,

l'imidazolinium

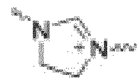

l'imidazolium :

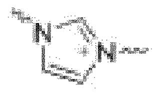

le triazolium :

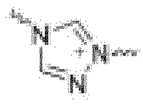

le pipérazinium :

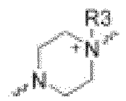

le pyridinium :

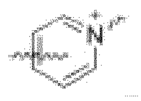

et
le triazinium :

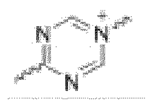

,

dans lesquels R3 représente $CH_{2r+1}$, r représentant un nombre entier de 1 à 4, R représentant des groupes alkyle ($C_1$ à $C_4$), m et n irrespectivement l'un de l'autre représentant un nombre entier de 1 à 6 et $AN^-$ représentant $-COO^-$ ou $-SO_3^-$, ledit capteur comprenant en outre un luminophore, comme détecteur d'un polluant, d'un biomarqueur ou d'un métabolite pharmaceutique.

Fig. 1 (Representative Figure)

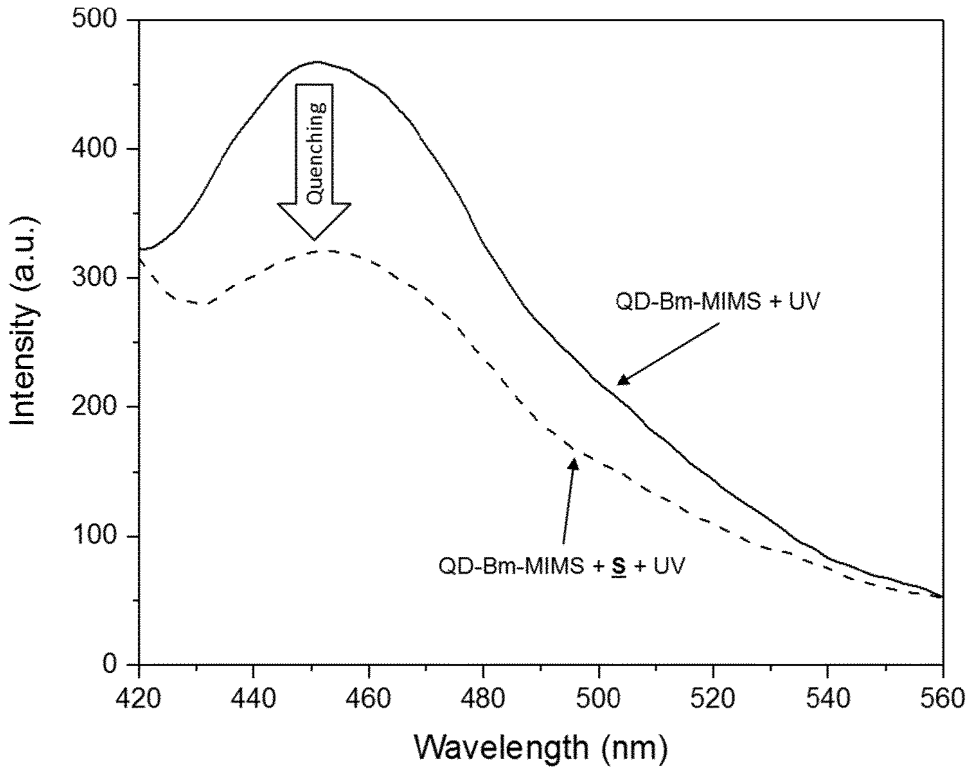

Fig. 2

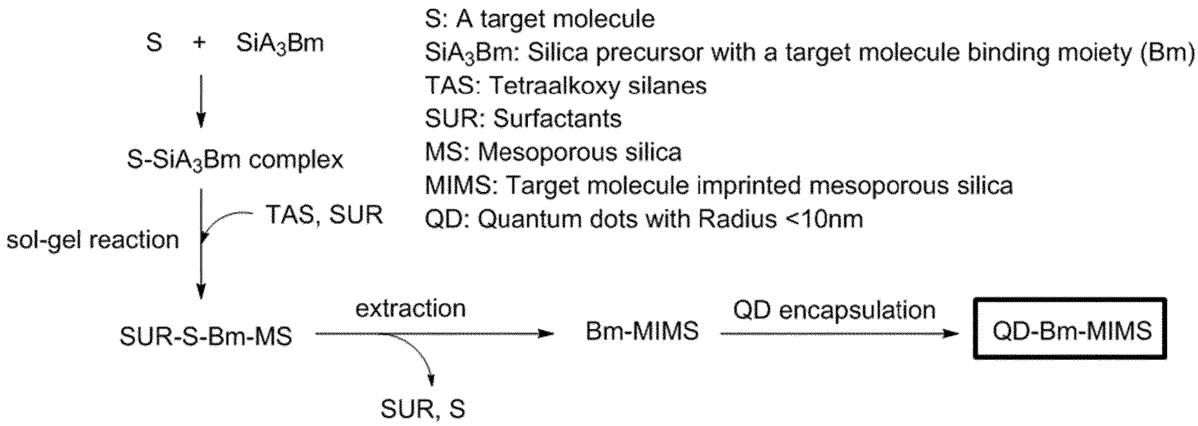
S + $SiA_3Bm$
$S-SiA_3Bm$ complex
sol-gel reaction
TAS, SUR
SUR-S-Bm-MS
extraction
SUR, S
Bm-MIMS
QD encapsulation
QD-Bm-MIMS
S: A target molecule
$SiA_3Bm$: Silica precursor with a target molecule binding moiety (Bm)
TAS: Tetraalkoxy silanes
SUR: Surfactants
MS: Mesoporous silica
MIMS: Target molecule imprinted mesoporous silica
QD: Quantum dots with Radius <10nm

**Fig. 3**

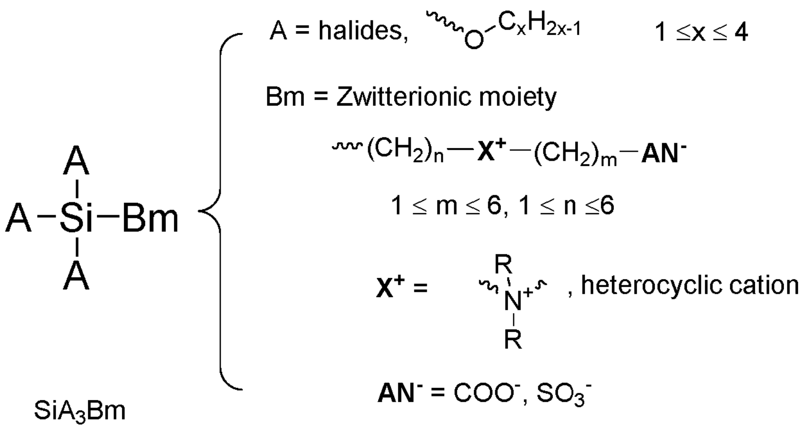


$SiA_3Bm$

Nitrogen hetero cyclic cation: pyrrolidinium, imidazolinium, imidazolium, triazolium, piperazinium, pyridium, triazinium,

R = $C_pH_{2p+1}$; R3 = $C_rH_{2r+1}$

1 ≤ p, r ≤ 4

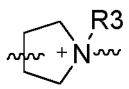
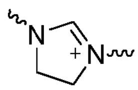
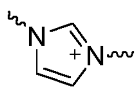
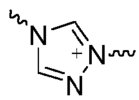

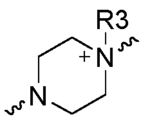
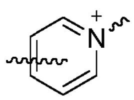
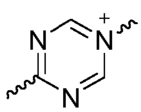

Fig. 4

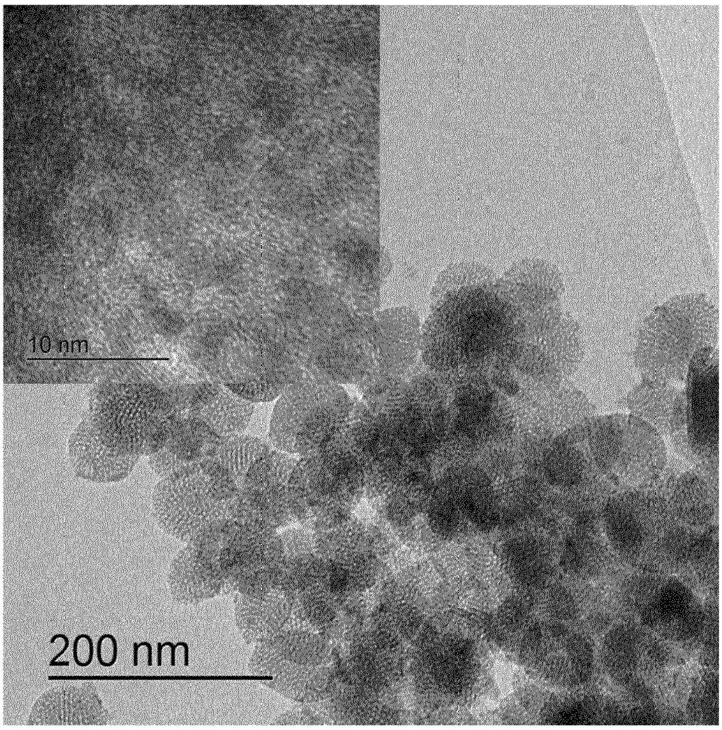


Fig. 5

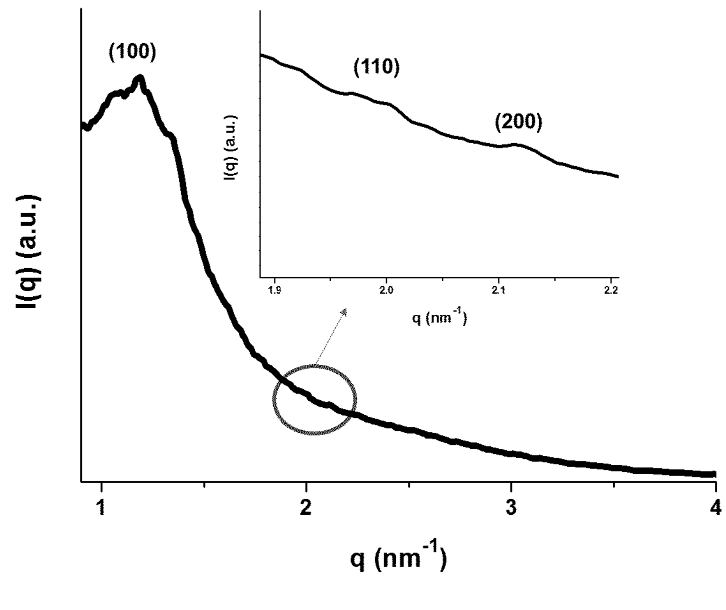

**Fig. 6**

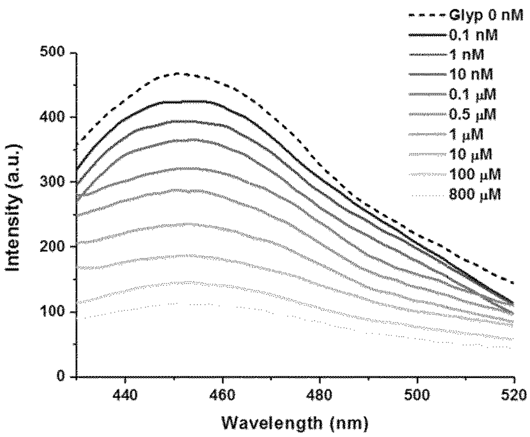
Glyp 0 nM
0.1 nM
1 nM
10 nM
0.1 μM
0.5 μM
1 μM
10 μM
100 μM
800 μM
Intensity (a.u.)
Wavelength (nm)

(a)

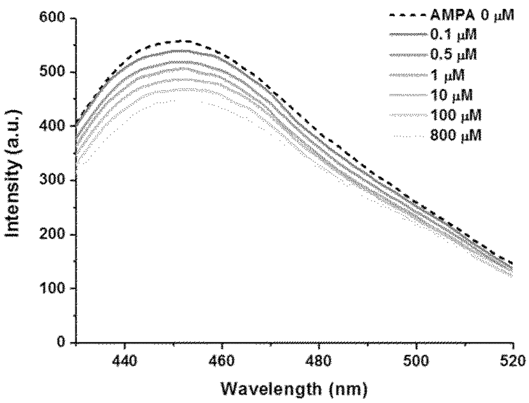
AMPA 0 μM
0.1 μM
0.5 μM
1 μM
10 μM
100 μM
800 μM
Intensity (a.u.)
Wavelength (nm)

(b)

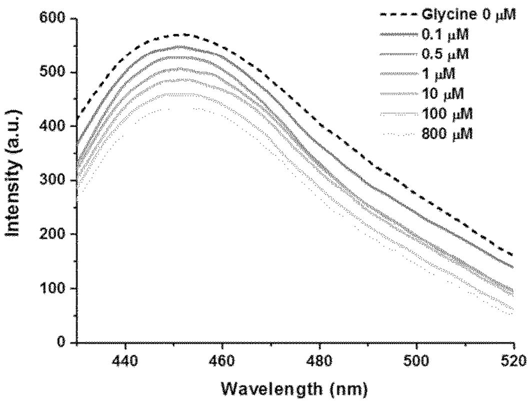
Glycine 0 μM
0.1 μM
0.5 μM
1 μM
10 μM
100 μM
800 μM
Intensity (a.u.)
Wavelength (nm)

(c)

**Fig. 7**

(a)

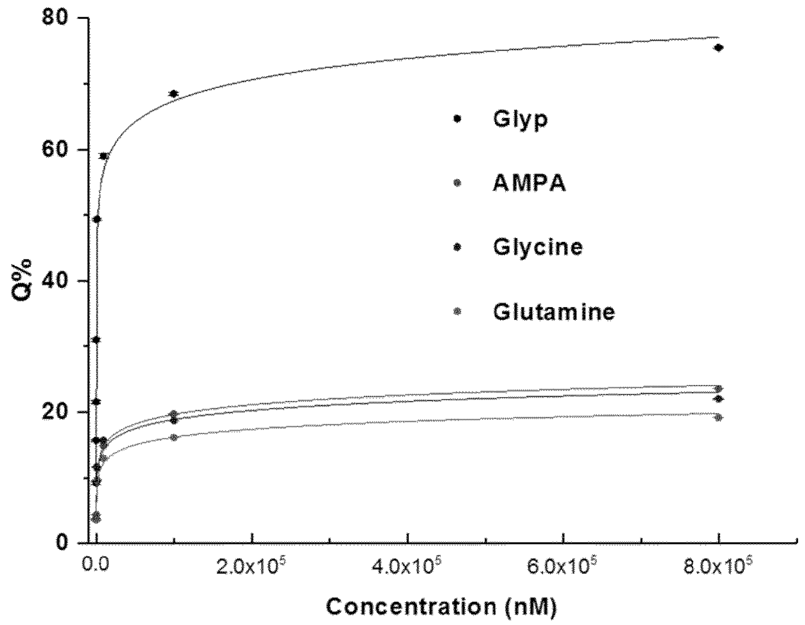
Q%
80
60
40
20
0
0.0
2.0x10^5
4.0x10^5
6.0x10^5
8.0x10^5
Concentration (nM)
Glyp
AMPA
Glycine
Glutamine

(b)

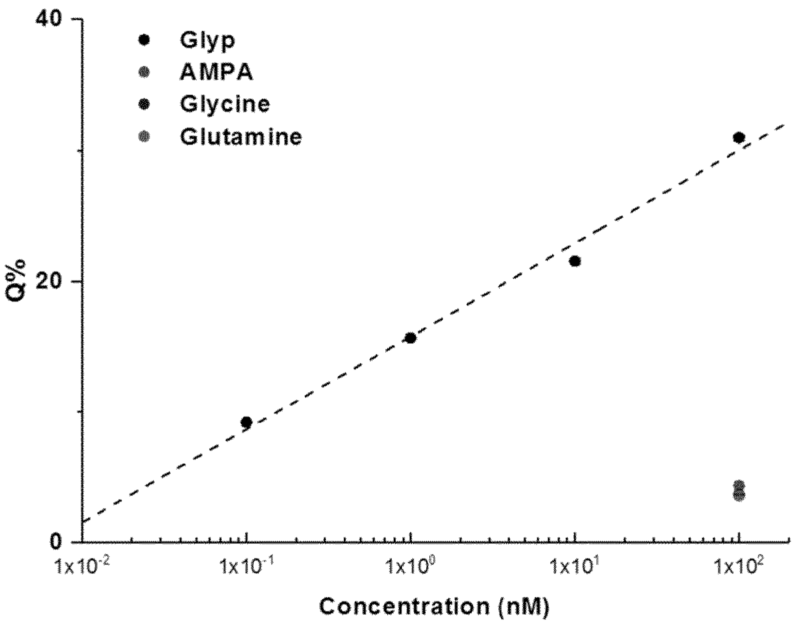
Q%
40
20
0
1x10^-2
1x10^-1
1x10^0
1x10^1
1x10^2
Concentration (nM)
Glyp
AMPA
Glycine
Glutamine

Fig. 8

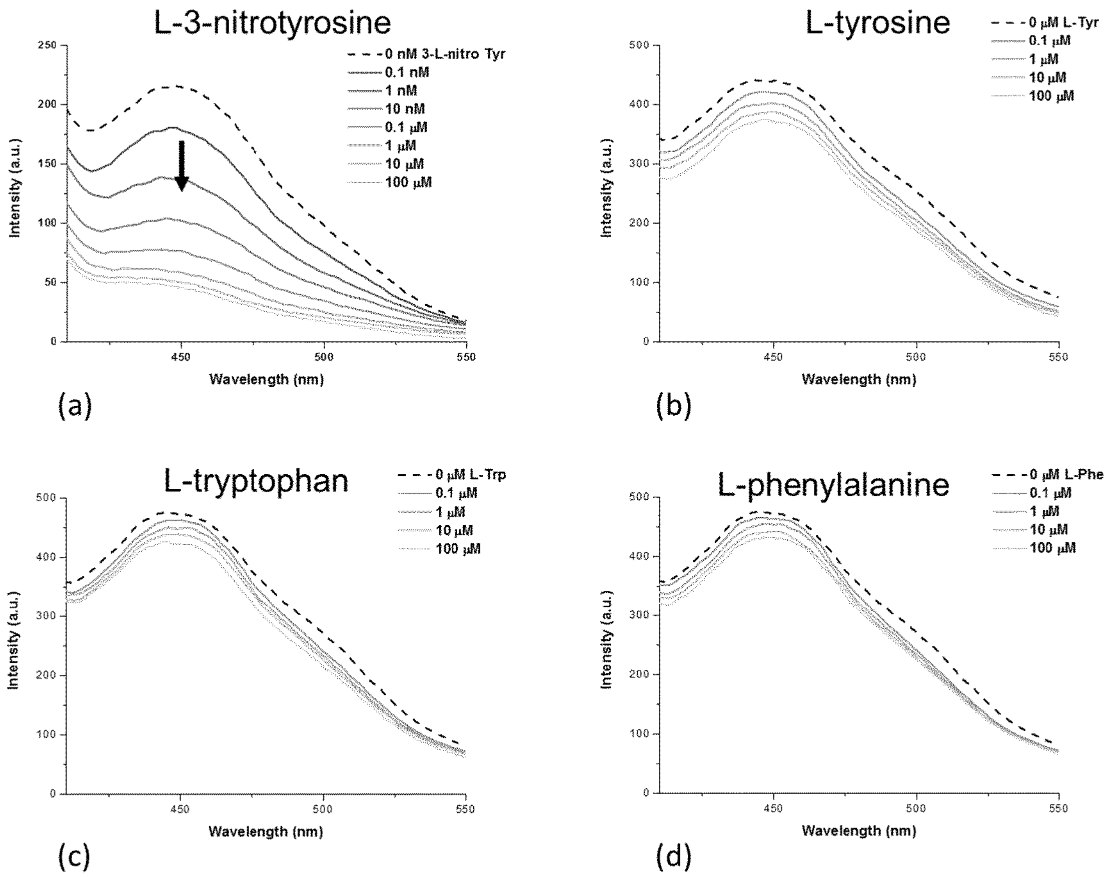
L-3-nitrotyrosine
0 nM 3-L-nitro Tyr
0.1 nM
1 nM
10 nM
0.1 μM
1 μM
10 μM
100 μM
Intensity (a.u.)
Wavelength (nm)
(a)
L-tyrosine
0 μM L-Tyr
0.1 μM
1 μM
10 μM
100 μM
Intensity (a.u.)
Wavelength (nm)
(b)
L-tryptophan
0 μM L-Trp
0.1 μM
1 μM
10 μM
100 μM
Intensity (a.u.)
Wavelength (nm)
(c)
L-phenylalanine
0 μM L-Phe
0.1 μM
1 μM
10 μM
100 μM
Intensity (a.u.)
Wavelength (nm)
(d)

Fig. 9

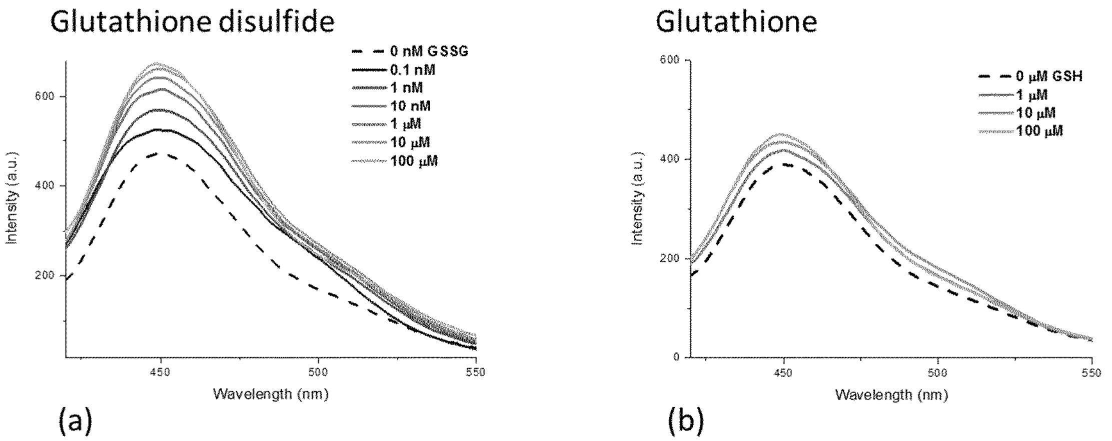
Glutathione disulfide
0 nM GSSG
0.1 nM
1 nM
10 nM
1 μM
10 μM
100 μM
Intensity (a.u.)
Wavelength (nm)
(a)
Glutathione
0 μM GSH
1 μM
10 μM
100 μM
Intensity (a.u.)
Wavelength (nm)
(b)

Fig. 10

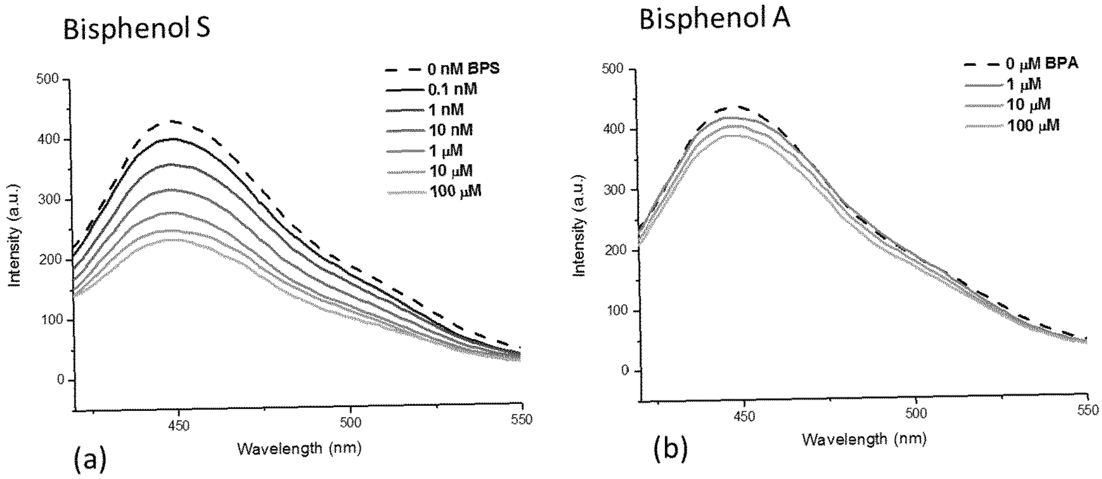


Fig. 11

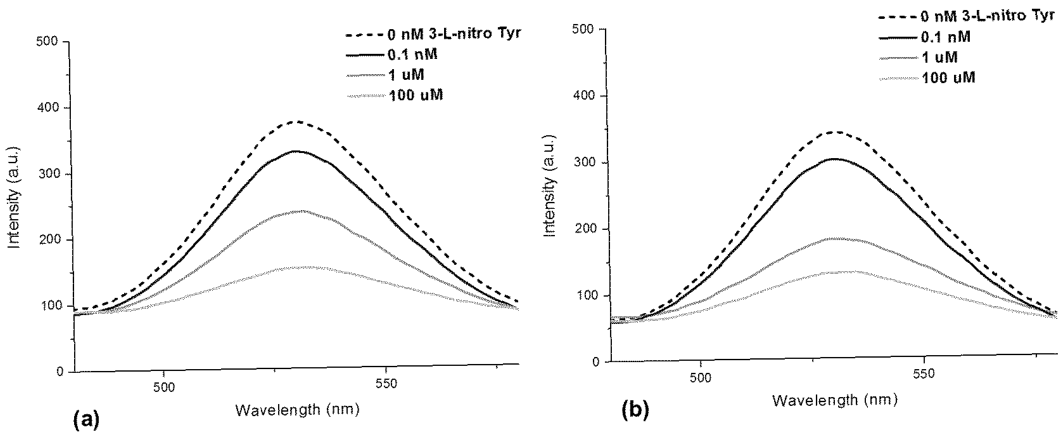


Fig. 12

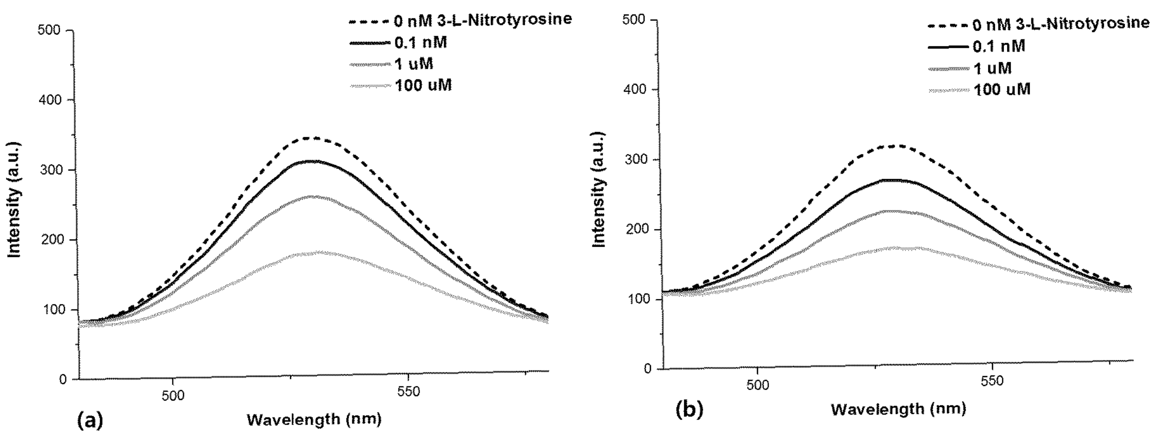

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 105601938 A


### Non-patent literature cited in the description


- **B. SELLERGREN et al.** Layer-by-layer grafting of molecularly imprinted polymers via iniferter modified supports. *Adv. Mater.*, 2002, vol. 14, 1204-1208
- **B. M. JUNG et al.** Molecularly imprinted mesoporous silica particles showing a rapid kinetic binding. *Chem. Commun.*, 2010, vol. 46, 3699-3701